# EUROPÄISCHE PATENTSCHRIFT

(11) **EP 0 471 262 B1**
(45) Veröffentlichungstag und Bekanntmachung des Hinweises auf die Patenterteilung: **15.11.1995**
(21) Anmeldenummer: 91113092.0
(22) Anmeldetag: 03.08.1991
(51) Int. Cl.: C07D 277/42, C07D 261/14, C07D 231/38, C07D 209/40, C07D 213/75, C07C 69/54, A01N 43/40, A01N 43/38, A01N 43/56, A01N 43/78, A01N 43/80, A01N 37/06

(54) **Verfahren zur Herstellung von substituierten 3-Aminoacrylestern**
Process for the production of substituted 3-amino-acrylesters
Procédé pour la production d'esters de 3-amino-acryl substitués

(30) Priorität: 16.08.1990 DE 4025892
(43) Veröffentlichungstag der Anmeldung: 19.02.1992
(73) Patentinhaber: BAYER AG, 51368 Leverkusen (DE)
(72) Erfinder: Gayer, Herbert, Dr., W-4019 Monheim (DE); Klausener, Alexander, Dr., W-4150 Krefeld 1 (DE); Knüppel, Peter Christian, Dr., W-5632 Wermelskirchen 3 (DE); Maurer, Fritz, Dr., Nihon Bayer Agrochem K.K., Yuki-shi, Ibaraki 307 (JP)

(56) Entgegenhaltungen:
- EP-A- 0 389 901
- DE-A- 3 805 059
- DE-A- 3 807 232

## Beschreibung

Die Erfindung betrifft ein neues Verfahren zur Herstellung von substituierten 3-Aminoacrylestern, welche als Fungizide oder als Zwischenprodukte zur Herstellung von fungiziden Alkoxyacrylestern verwendet werden können.

Es ist bekannt, daß man bestimmte fungizid wirksame Alkoxyacrylester erhält, wenn man geeignete Essigsäureesterderivate mit Ameisensäureesterderivaten in Gegenwart einer Base kondensiert und dann die so erhältlichen 3-Hydroxyacrylesterderivate in einer zweiten Stufe mit Alkylierungsmitteln in Gegenwart einer Base an der 3-Hydroxygruppe alkyliert (vgl. z.B. DE-OS 3805059; DE-OS 3807232).

Der Nachteil dieses Verfahrens besteht darin, daß die als erste Stufe durchgeführte Claisen-Kondensation häufig nur sehr schlechte Ausbeuten liefert und bei einigen Essigsäureesterderivaten in Abhängigkeit von der Art des Substituenten in der 2-Position ganz versagt.

Weiterhin ist bekannt, daß man fungizid wirksame substituierte 3-Aminoacrylester erhält, wenn man geeignete Essigsäureesterderivate mit Formamiden oder Formamidderivaten umsetzt (vgl. z.B. DE-OS 3807232; DE-OS 3910358). Die dabei verwendeten Dimethylformamiddialkylacetale liefern jedoch ebenfalls häufig nur sehr geringe Ausbeuten an gewünschtem Endprodukt.

Es wurde nun gefunden, daß man substituierte 3-Aminoacrylester der allgemeinen Formel (I), 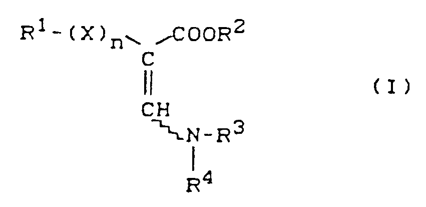
in welcher
- R¹: für einen gegebenenfalls einfach oder mehrfach, gleich oder verschieden substituierten, gesättigten, einfach oder mehrfach ungesättigten oder aromatischen Carbocyclus mit 5 bis 10 Kohlenstoffatomen oder für einen gegebenenfalls einfach oder mehrfach, gleich oder verschieden substituierten, gesättigten, einfach oder mehrfach ungesättigten oder aromatischen Heterocyclus mit 2 bis 9 Kohlenstoffatomen und 1 bis 5 gleichen oder verschiedenen Heteroatomen - insbesondere Stickstoff, Sauerstoff oder Schwefel - steht, wobei als Substituenten jeweils infrage kommen;
Halogen, Cyano, Nitro, jeweils geradkettiges oder verzweigtes Alkyl, Alkoxy oder Alkylthio mit jeweils 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl, Alkoximinoalkyl, Dialkylamino oder Dialkylaminocarbonyl mit jeweils 1 bis 4 Kohlenstoffatomen in den einzelnen Alkylteilen, jeweils geradkettiges oder verzweigtes Alkenyl oder Alkinyl mit jeweils 2 bis 8 Kohlenstoffatomen, Cycloalkyl mit 3 bis 7 Kohlenstoffatomen, zweifach verknüpftes Alkandiyl mit 3 bis 5 Kohlenstoffatoman, oder jeweils im Arylteil oder im Heteroarylteil gegebenenfalls einfach oder mehrfach, gleich oder verschieden
- durch Halogen, Alkyl, Alkoxy, Alkylthio, Halogenalkyl, Halogenalkoxy, Halogenalkylthio, Dioxyalkylen, Halogen-substituiertes Dioxyalkylen oder gegebenenfalls substituiertes Phenyl
substituiertes Aryl, Aryloxy, Arylthio, Arylcarbonyl, Aralkyl, Aralkenyl, Aralkinyl, Aralkyloxy, Aralkylthio, Heteroarylalkyl, Heteroarylalkenyl, Heteroaryloxy, Heteroarylthio, Heteroarylcarbonyl, Heteroarylalkyloxy, Heteroarylalkylthio oder Heteroaryl mit jeweils 6 bis 10 Kohlenstoffatomen im Arylteil oder mit 2 bis 9 Kohlenstoffatomen und 1 bis 4 gleichen oder verschiedenen Heteroatomen - insbesondere Stickstoff, Sauerstoff und/oder Schwefel - im Heteroarylteil und gegebenenfalls 1 bis 6 Kohlenstoffatomen im geradkettigen oder verzweigten Alkylteil oder gegebenenfalls 2 bis 6 Kohlenstoffatomen im geradkettigen oder verzweigten Alkenylteil bzw. Alkinylteil;
- R²: für geradkettiges oder verzweigtes Alkyl mit 1 bis 6 Kohlenstoffatomen oder für gegebenenfalls im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten infrage kommen; Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 9 Kohlenstoffatomen und 1 bis 4 gleichen oder verschiedenen Heteroatomen - insbesondere Stickstoff, Sauerstoff und/oder Schwefel - im Heteroarylteil und gegebenenfalls 1 bis 4 Kohlenstoffatomen im geradkettigen oder verzweigten Alkylteil;
- R³ und R⁴: entweder unabbängig voneinander jeweils für geradkettiges oder verzweigtes Alkyl mit 1 bis 6 Kohlenstoffatomen stehen oder gemeinsam mit dem Stickstoffatom, an welches sie gebunden sind, für einen gegebenenfalls einfach oder mehrfach, gleich oder verschieden substituierten, gesättigten 5- bis 7-gliedrigen Heterocyclus stehen, der gegebenenfalls ein weiteres Heteroatom - insbesondere Stickstoff, Sauerstoff oder Schwefel - enthalten kann, wobei als Substituenten geradkettige oder verzweigte Alkylreste mit 1 bis 4 Kohlenstoffatomen infrage kommen,
- X: für Sauerstoff, Schwefel oder für einen der Reste 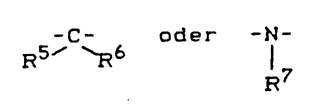 steht und
- n: für eine Zahl 0 oder 1 steht, wobei
- R⁵, R⁶ und R⁷: unabhängig voneinander jeweils für Wasserstoff, für jeweils geradkettiges oder verzweigtes Alkyl mit 1 bis 6 Kohlenstoffatomen, Alkenyl mit 2 bis 6 Kohlenstoffatomen oder für jeweils gegebenenfalls im Arylteil einfach oder mehrfach, gleich oder verschieden substituiertes Aralkyl mit 1 bis 6 Kohlenstoffatomen im geradkettigen oder verzweigten Alkylteil und 6 bis 10 Kohlenstoffatomen im Arylteil oder Aralkenyl mit 2 bis 6 Kohlenstoffatomen im geradkettigen oder verzweigten Alkenylteil und 6 bis 10 Kohlenstoffatomen im Arylteil stehen, wobei als Arylsubstituenten jeweils die bei R² genannten infrage kommen,
erhält, wenn man substituierte Essigsäureester der Formel (II),

R¹-(X)ₙ-CH₂-COOR² (II)

in welcher
R¹, R², X und n die oben angegebene Bedeutung haben,
mit Orthoameisensäurediamidestern der Formel (III), 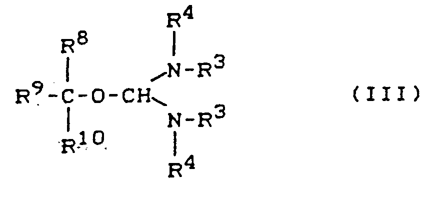
in welcher
- R⁸: für Wasserstoff oder Alkyl steht,
- R⁹ und R¹⁰: entweder unabhängig voneinander jeweils für Alkyl stehen oder gemeinsam mit dem Kohlenstoffatom, an welches sie gebunden sind, für einen Cycloalkylrest stehen und
- R³ und R⁴: die oben angegebene Bedeutung haben,
gegebenenfalls in Gegenwart eines Verdünnungsmittels umsetzt.

Dabei ist es ausgesprochen überraschend, daß die erfindungsgemäße Umsetzung mit Orthoameisensäurediamidestern hohe Ausbeuten an gewünschten Endprodukten erbringt, da die Umsetzungen mit vergleichbar reaktiven Dimethylformamiddialkylacetalen in vielen Fällen nicht durchführbar sind.

Daher ist es ein besonderer Vorteil des erfindungsgemäßen Verfahrens, daß mit seiner Anwendung Verbindungen zugänglich sind, die vorher gar nicht oder nur in sehr geringer Ausbeute zu erhalten waren.

Ein weiterer Vorteil des erfindungsgemäßen Verfahrens besteht darin, daß sich die mit Hilfe des erfindungsgemäßen Verfahrens einfach und in hoher Ausbeute erhältlichen substituierten 3-Aminoacrylester auch als Zwischenprodukte zur Herstellung von fungiziden 3-Alkoxyacrylestern eignen, wenn man die 3-Aminogruppe sauer hydrolysiert und die so erhältlichen 3-Hydroxyacrylester entweder in einer zweiten separaten Reaktionsstufe oder direkt anschließend im Eintopfverfahren alkyliert. Die als Fungizide im Pflanzenschutz wertvollen 3-Alkoxyacrylester können somit einfacher und in besseren Ausbeuten hergestellt werden, als mit den bekannten Verfahren.

Die mit Hilfe des erfindungsgemäßen Verfahrens erhältlichen substituierten 3-Aminoacrylester sind durch die Formel (I) allgemein definiert, Bevorzugt herstellbar sind Verbindungen der allgemeinen Formel (I), bei welchen
- R¹: für einen gegebenenfalls ein- bis fünffach, gleich oder verschieden substituierten und/oder benzannellierten Phenylrest oder für einen gegebenenfalls ein- bis fünffach, gleich oder verschieden substituierten Cycloalkenylrest mit 5 bis 7 Kohlenstoffatomen oder für einen gegebenenfalls ein- bis fünffach, gleich oder verschieden substituierten und/oder benzannellierten Heteroarylrest mit 1 bis 5 Kohlenstoffatomen und 1 bis 3 Heteroatomen - insbesondere Stickstoff, Sauerstoff oder Schwefel - steht, wobei als Substituenten jeweils infrage kommen;
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl, Dimethylamino, Diethylamino, Dimethylcarbamoyl, Diethylcarbamoyl, Allyl, Butenyl oder Propargyl, Cyclopropyl, Cyclopentyl, Cyclohexyl, 1,3-Propandiyl, 1,4-Butandiyl, 1,5-Pentandiyl oder jeweils gegebenenfalls ein- bis dreifach, gleich oder verschieden durch Fluor, Chlor, Brom, Methyl, Ethyl, n- oder i-Propyl, n-, i-, s- oder t-Butyl, Methoxy, Ethoxy, n- oder i-Propoxy, Methylthio, Ethylthio, Trifluormethyl, Trifluormethoxy, Trifluormethylthio, Dioxymethylen oder gegebenenfalls ein- bis dreifach, gleich oder verschieden durch Fluor, Chlor, Methyl, Methoxy oder Trifluormethyl substituiertes Phenyl, substituiertes Phenyl, Naphthyl, Phenoxy, Phenylthio, Phenylcarbonyl, Benzyl, Phenylethyl, Phenylpropyl, Phenylethenyl, Benzyloxy, Heteroaryloxy, Heteroarylmethyl oder Heteroaryl, wobei als Heteroarylreste im einzelnen jeweils die folgenden infrage kommen: 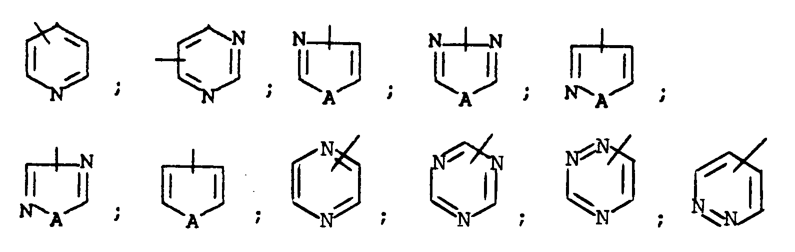 welche gegebenenfalls auch benzannelliert sein können und bei welchen
- A: jeweils für Sauerstoff, Schwefel oder eine NH-Gruppe steht;
- R²: für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy oder Benzyloxy,
- R³ und R⁴: entweder unabhängig voneinander jeweils für Methyl, Ethyl, n- oder i-Propyl, n-, i-, s- oder t-Butyl stehen oder gemeinsam mit dem Stickstoffatom, an welches sie gebunden sind, für einen 1-Pyrrolidinyl-, 1-Piperidinyl- oder 4-Morpholinylrest stehen,
- X: für Sauerstoff, Schwefel oder für einen der Reste 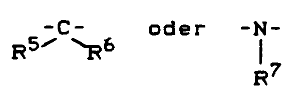 steht und
- n: für eine Zahl 0 oder 1 steht, wobei
- R⁵, R⁶ und R⁷: unabhängig voneinander jeweils für Wasserstoff, Methyl, Ethyl, n- oder i-Propyl, n-, i-, s- oder t-Butyl oder für jeweils gegebenenfalls einbis dreifach, gleich oder verschieden substituiertes Benzyl oder Phenyl stehen, wobei als Substituenten jeweils die bei R² genannten infrage kommen.

Ganz besonders bevorzugt herstellbar sind Verbindungen der allgemeinen Formel (I), bei welchen
- R¹: für einen gegebenenfalls ein- bis dreifach substituierten und/oder benzannellierten Phenylrest, für einen jeweils gegebenenfalls ein- bis dreifach, gleich oder verschieden substituierten und/oder benzannellierten Cyclohexenylrest oder Cyclopentenylrest oder für einen gegebenenfalls ein- bis dreifach substituierten und/oder benzannellierten Heteroarylrest steht, wobei als Heteroarylreste insbesondere infrage kommen: 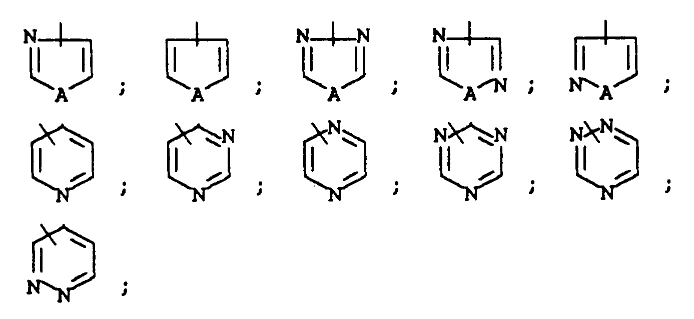
wobei
- A: jeweils für Sauerstoff, Schwefel oder eine NH-Gruppe steht und wobei als Phenyl-, Cyclopentenyl-, Cyclohexenyl- oder Heteroarylsubstituenten jeweils infrage kommen:
Fluor, Chlor, Brom, Cyano, Nitro, Methyl, Ethyl, Methoxy, Methylthio, Trifluormethyl, Trifluormethoxy, Trifluormethylthio, Methoxycarbonyl, Ethoxycarbonyl oder Dimethylamino oder jeweils gegebenenfalls ein- bis dreifach, gleich oder verschieden durch Fluor, Chlor, Methyl, Ethyl, Methoxy, Trifluormethyl, Dioxymethylen oder Phenyl substituiertes und/oder benzannelliertes Phenyl, Phenoxy, Phenylcarbonyl, Benzyl, Pyridyl, Pyrazolyl, Oxazolyl, Thiazolyl, Thienyl, Furyl, Thiadiazolyl, Oxadiazolyl, Imidazolyl oder Triazolyl,
- R²: für Methyl, Ethyl, n- oder i-Propyl oder Benzyl steht,
- R³ und R⁴: jeweils für Methyl oder Ethyl stehen,
- X: für Sauerstoff, Schwefel oder für einen der Reste 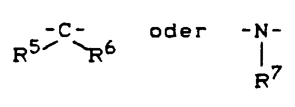 steht und
- n: für eine Zahl 0 oder 1 steht, wobei
- R⁵, R⁶ und R⁷: unabhängig voneinander jeweils für Wasserstoff, Methyl, Ethyl, n- oder i-Propyl oder Benzyl stehen.

Aryl als solches oder in Zusammensetzungen bedeutet Phenyl oder Naphthyl, insbesondere Phenyl.

Alle aliphatischen Reste als solche oder in Zusammensetzungen sind geradkettig oder verzweigt.

Halogen steht, wann nicht anders definiert, für Fluor, Chlor, Brom oder Iod, insbesondere für Fluor, Chlor oder Brom.

Verwendet man beispielsweise N-Methyl-N-[2-(6-phenyl)-pyridyl]-glycinmethylester und 1,1-Bis-[dimethylamino]-methyl-t-butylether als Ausgangsstoffe, so läßt sich der Reaktionsablauf des erfindungsgemäßen Verfahrens durch das folgende Formelschema darstellen: 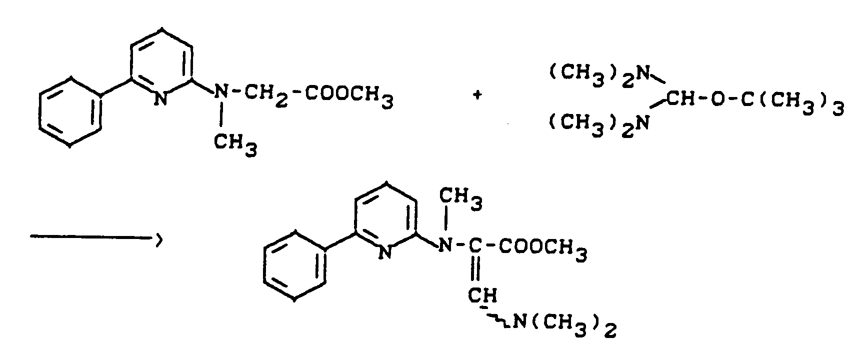
Die zur Durchführung des erfindungsgemäßen Verfahrens als Ausgangsstoffe benötigten substituierten Essigsäureester sind durch die Formel (II) allgemein definiert. In dieser Formel (II) stehen R¹, R², X und n vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Essigsäureester der Formel (II) sind bekannt oder erhältlich in Analogie zu bekannten Verfahren (vgl. z.B. J. Chem. Pharm. Bull. 23, 3008-3010 [1975]; Prakt. Chem. 315, 1175-1182 [1973]; Chimia 28, 235-236 [1974]; Pak. J. Sci. Ind. Res. 20, 139-149 [1977]; J. Heterocycl. Chem. 5, 281-283 [1968]; Pol. J. Chem. 53, 2349-2354 [1979]; J. Heterocycl. Chem. 24, 85-89 [1987]; Zh. org. Khim. 20, 1517-1538 [1984]; Zh. Org. Khim. 20, 2002-2011 [1984]; Izv. Akad. Nauk SSSR. Ser. Khim. 1984, 2760-2765; DE 21 03 728; DE 26 37 911; DE 27 09 108; DE 27 25 361; DE 24 25 282; GB 11 61 492 [1969]; EP 182 769; EP 245 230; EP 227 932; DE-OS 3904931; DE-OS 3805059; DE-OS 3807232; DE-OS 3905119; DE-OS 3904931).

Die zur Durchführung des erfindungsgemäßen Verfahrens weiterhin als Ausgangsstoffe benötigten Orthoameisensäurediamidester sind durch die Formel (III) allgemein definiert. In dieser Formel (III) stehen R³ und R⁴ vorzugsweise für diejenigen Reste, die bereits im Zusammenhang mit der Beschreibung der erfindungsgemäßen Stoffe der Formel (I) als bevorzugt für diese Substituenten genannt wurden.
- R⁸: steht vorzugsweise für Wasserstoff oder für geradkettiges oder verzweigtes Alkyl mit 1 bis 4 Kohlenstoffatomen, besonders bevorzugt für Wasserstoff oder Methyl;
- R⁹ und R¹⁰: stehen vorzugsweise unabhängig voneinander jeweils für geradkettiges oder verzweigtes Alkyl mit 1 bis 4 Kohlenstoffatomen oder gemeinsam mit dem Kohlenstoffatom, an welches sie gebunden sind, für Cycloalkyl mit 5 bis 7 Kohlanstoffatomen;
- R⁹ und R¹⁰: stehen besonders bevorzugt für Methyl oder Ethyl oder gemeinsam mit dem Kohlenstoffatom, an welches sie gebunden sind, für einen Cyclohexylrest.

Die Orthoameisensäurediamidester der Formel (III) sind ebenfalls bekannt (vgl. z.B. Chem. Bar. 101, 41-50 [1968]; Chem. Ber. 101, 1885-1888 [1968]; DE 2303919; PCT Int. Appl. WO 8601204) oder erhältlich in Analogie zu bekannten Verfahren.

Als Verdünnungsmittel zur Durchführung das erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl- oder -diethylether, Amide wie Dimethylformamid, Dimethylacetamid, N-Methylformanilid, N-Methylpyrrolidon oder Hexamethylphosphorsäuretriamid,

Es ist auch möglich, das erfindungsgemäße Verfahren ganz ohne Zusatz von Lösungsmitteln durchzuführen.

Die Reaktionstemperaturen können bei der Durchführung des erfindungsgemäßen Verfahrens in einem größeren Bereich variiert werden. Im allgemeinen arbeitet man bei Temperaturen von -35°C bis +150°C, vorzugsweise bei Temperaturen von 0°C bis 120°C.

Das erfindungsgemäße Verfahren kann auch unter vermindertem oder erhöhtem Druck durchgeführt werden, bevorzugt arbeitet man jedoch unter Normaldruck.

Gegebenenfalls kann dar Einsatz einer Inertgasatmosphäre wie beispielsweise Stickstoff oder Argon zweckmäßig sein, im allgemeinen ist es jedoch möglich, das erfindungsgemäße Verfahren unter normaler Raumluftatmosphäre durchzuführen.

Zur Durchführung des erfindungsgemäßen Verfahrens setzt man pro Mol an substituiertem Essigsäureester der Formel (II) im allgemeinen 1.0 bis 15.0 Mol, vorzugsweise 1.0 bis 5.0 Mol an Orthoameisensäurediamidester der Formel (III) ein.
Die Reaktionsdurchführung, Aufarbeitung und Isolierung der Reaktionsprodukte erfolgt nach allgemein üblichen Methoden (vgl. auch die Herstellungsbeispiele).

Die mit Hilfe des erfindungsgemäßen Verfahrens herstellbaren substituierten 3-Aminoacrylester sind teilweise bekannte Fungizide (vgl. z.B. DE-OS 3904931; DE-OS 3905119; DE-OS 3807232). Die neuen 3-Aminoacrylester werden in einer parallelen Anmeldung beansprucht.

Darüber hinaus lassen sie sich als Zwischenprodukte verwenden zur Herstellung von weiteren bekannten Fungiziden der Formel (IV), 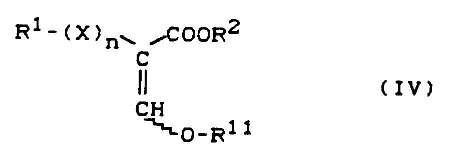
in welcher
R¹¹ für Alkyl steht und
R¹, R², X und n die oben angegebene Bedeutung haben,
(vgl. z.B. DE-OS 3904931; DE-OS 3905119; DE-OS 3807232), indem man in üblicher Art und Weise, beispielsweise mit äquimolaren Mengen an verdünnter Salzsäure bei Temperaturen zwischen 0° und 60°C gegebenenfalls in Gegenwart eines Verdünnungsmittels wie beispielsweise Acetonitril oder Dimethylformamid innerhalb von vier bis acht Stunden zunächst die Aminogruppe in 3-Position hydrolysiert (bei Ausgangsverbindungen der Formel (I), welche mehrere basische Gruppen im Molekül enthalten, ist es hierbei erforderlich, für jede basische Gruppe ein Äquivalent Salzsäure einzusetzen) und anschließend entweder in einer getrennten Reaktionsstufe oder im Eintopfverfahren die so erhältlichan substituierten 3-Hydroxyacrylester der Formel (V), 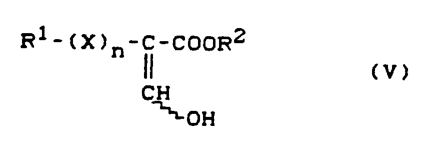
in welcher
R¹, R², X und n die oben angegebene Bedeutung haben,
ebenfalls in üblicher Art und Weise mit Alkylierungsmitteln, wie beispielsweise Dimethylsulfat gegebenenfalls in Gegenwart eines Verdünnungsmittels wie beispielsweise Dimethylformamid und gegebenenfalls in Gegenwart eines Reaktionshilfsmittels wie beispielsweise Kaliumcarbonat bei Temperaturen zwischen -20° und +120°C alkyliert.

### Herstellungsbeispiele:

### Beispiel 1:

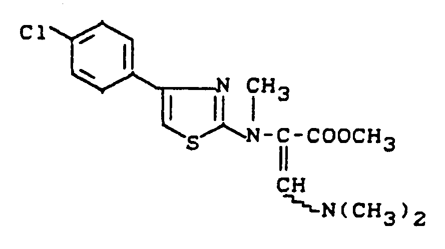

Ein Gemisch aus 14.8 g (0.04987 Mol) N-[4-(4-Chlorphenyl)-thiazol-2-yl)-N-methylaminoessigsäuremethylester (Herstellung analog DE-OS 3807232) und 20 g (0.124 Mol) t-Butyloxy-bis-(dimethylamino)-methan wird 12 Stunden auf 100°C erwärmt, anschließend abgekühlt, mit 200 ml Wasser und 200 ml Ethylacetat versetzt, 2 Stunden bei Raumtemperatur gerührt und ausgefallene Kristalle abgesaugt und getrocknet. Die Ethylacetatphase wird eingeengt, der Rückstand mit Diisopropylether aufgeschlämmt, abgesaugt und getrocknet.

Man erhält 16.3 g (92.9% der Theorie) an 2-{N-[4-(4-Chlorphenyl)-thiazol-2-yl)-N-methyl-amino}-3-dimethylaminoacrylsäuremethylester vom Schmelzpunkt 177-178°C (Reinheit 99% laut Gaschromatographie).

### Beispiel IV-1:

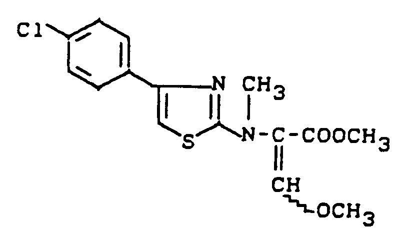

Zu 5.3 g (0.015 Mol 2-{N-[4-(4-Chlorphenyl)-thiazol-2-yl)-N-methylamino}-3-dimethylaminoacrylsäuremethylester in 20 ml Dimethylformamid gibt man 15 ml (0.033 Mol) 2 normale wäßrige Salzsäure, erwärmt für 4 Stunden auf 60°C, kühlt ab, gießt die Mischung in gesättigte wäßrige Ammoniumchloridlösung und extrahiert mit Ethylacetat. Die organische Phase wird eingeengt, der Rückstand in 30 ml Dimethylformamid gelöst, mit 2.05 g (0.015 Mol) Kaliumcarbonat und 3.05 g (0.023 Mol) Dimethylsulfat versetzt und 16 Stunden bei Raumtemperatur gerührt. Zur Aufarbeitung gibt man die Reaktionsmischung in Wasser, extrahiert mit Ethylacetat, trocknet die organische Phase über Natriumsulfat, engt im Vakuum ein und chromatographiert den Rückstand an Kieselgel (Laufmittel: Ethylacetat/Hexan).

Man erhält 4.6 g (90% der Theorie) an 2-{N-[4-(4-Chlorphenyl)-thiazol-2-yl]-N-methylamino}-3-methoxyacrylsäuremethylester vom Schmelzpunkt 104-105°C.

### Beispiel 2:

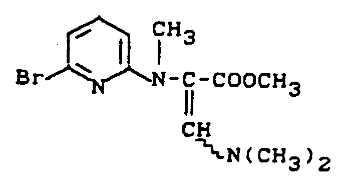

1.05 g (0.00405 Mol) [N-(6-Brom-2-pyridyl)-N-methyl]-aminoessigsäuremethylester (vgl. z.B. DE-OS 3904931) und 1.05 g (0.00651 Mol) t-Butyloxy-bis-(dimethylamino)-methan werden in 10 ml Toluol 18 Stunden auf Rückflußtemperatur erhitzt. Zur Vervollständigung der Umsetzung gibt man weitere 0.5 g (0.0031 Mol) t-Butyloxy-bis-(dimethylamino)-methan zu, erwärmt für 2 weitere Stunden auf Rückflußtemperatur, kühlt dann ab, gibt Wasser zu und rührt 2 Stunden bei Raumtemperatur. Anschließend wird die organische Phase abgetrennt, die wäßrige Phase mit Toluol extrahiert, die vereinigten organischen Extrakte über Natriumsulfat getrocknet und eingeengt.

Man erhält 1.15 g (90.4% der Theorie) an 2-[N-(6-Brom-2-pyridyl)-N-methyl]-amino-3-dimethylaminoacrylsäuremethylester vom Schmelzpunkt 106°C (Reinheit 90% laut Gaschromatographie).

### Beispiel 3:

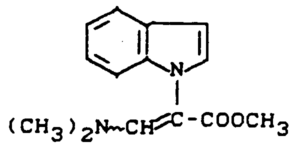

7.6 g (0.04 Mol) 1-Indolylessigsäuremethylester und 10.5 g (0.06 Mol) t-Butyloxy-bis-(dimethylamino)-methan werden bei einer Badtemperatur von 100°C eine Stunde erhitzt. Anschließend entfernt man flüchtige Bestandteile im Vakuum und chromatographiert den Rückstand an Kieselgel (Laufmittel: Dichlormethan).

Man erhält 6.5 g (67% der Theorie) an 3-Dimethylamino-2-indolylacrylsäuremethylester als Öl.

¹H-NMR (CDCl₃/Tetramethylsilan): δ = 6.57 (1H); 7.0 (1H); 7.1-7.2 (3H); 7.55-7.65 (1H); 7.7 (1H) ppm.

### Beispiel 4:

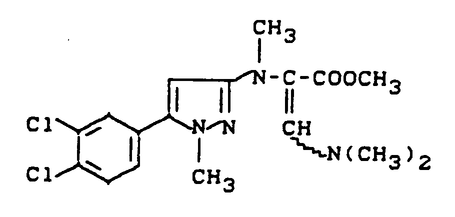

3.3 g (0.01 Mol) N-[5-(3,4-Dichlorphenyl)-1-methylpyrazol-3-yl]-N-methylaminoessigsäuremathylester werden zusammen mit 2.6 g (0.015 Mol) t-Butoxy-bis-(dimethylamino)-methan bei einer Badtemperatur von 100°C eine Stunde lang erhitzt. Das so erhältliche Reaktionsgemisch wird durch Chromatographie an Kieselgel (Laufmittel: Ether/Petrolether 1:3) aufgetrennt.

Man erhält 2.7 g (71% der Theorie) an 2-{N-[5-(3,4-Dichlorphenyl)-1-methyl-pyrazol-3-yl]-N-methylamino}-3-dimethylaminoacrylsäuremethylester vom Schmelzpunkt 113-114°C.

### Beispiel V-4

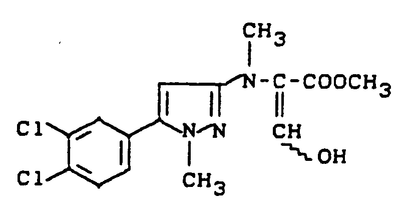

3 g (0.0078 Mol) 2-{N-[5-(3,4-Dichlorphenyl)-1-methylpyrazol-3-yl]-N-methylamino}-3-dimethylaminoacrylsäuremethylester werden mit 4.3 ml (0.0086 Mol) 2 normaler Salzsäure 6 Stunden lang auf 60°C erwärmt. Zur Aufarbeitung verdünnt man das Reaktionsgemisch mit Wasser, extrahiert mit Dichlormethan, trocknet die organische Phase über Natriumsulfat und entfernt anschließend das Lösungsmittel im Vakuum.

Man erhält 1.3 g (47% der Theorie) an 2-{N-[5-(3,4-Dichlorphenyl)-1-methyl-pyrazol-3-yl]-N-methylamino}-3-hydroxyacrylsäuremethylester als öliges Rohprodukt, welches ohne weitere Reinigung in die nächste Stufe eingesetzt werden kann.

### Beispiel IV-4

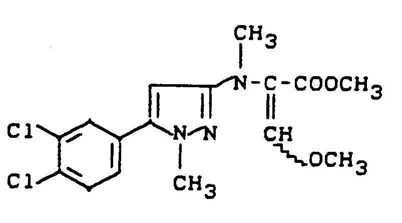

1.3 g (0.0036 Mol) 2-{N-[5-(3,4-Dichlorphenyl)-1-methylpyrazol-3-yl]-N-methylamino}-3-hydroxyacrylsäuremethylester und 1 g (0.0072 Mol) Kaliumcarbonat werden zusammen mit 0.51 g (0.004 Mol) Dimethylsulfat in 5 ml Dimethylformamid 16 Stunden bei Raumtemperatur gerührt. Zur Aufarbeitung wird im Vakuum eingeengt, der Rückstand zwischen Wasser und Dichlormethan verteilt, die organische Phase abgetrennt, getrocknet, eingeengt und der Rückstand an Kieselgel chromatographiert. (Laufmittel: Dichlormethan/Methanol 40:1).

Man erhält 0.9 g (67% der Theorie) an 2-{N-[5-(3,4-Dichlorphenyl)-1-methylpyrazol-3-yl)-N-methylamino}-methoxyacrylsäuremethylester als Öl.

¹H-NMR (CDCl₃/Tetramethylsilan): δ = 7.86 (1H); 7.57-7.6 (1H); 7.39, 7.42 (d, 1H); 7.25 (1H); 6.07 (1H); 3.86 (3H); 3.70 (3H); 3.63 (3H); 3.05 (3H) ppm.

### Beispiel II-4:

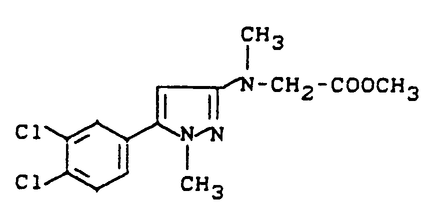

Zu 81 g (0.3 Mol) (3,4-Dichlorphenyl)-(2,2-dichlorvinyl)-keton (vgl. Synthesis 1982, 204) in 300 ml Tetrahydrofuran gibt man bei Raumtemperatur tropfenweise unter Rühren eine Mischung aus 30.3 g (0.3 Mol) Triethylamin und 30.9 g (0.3 Mol) Sarcosinmethylester zu und rührt anschließend 16 Stunden bei Raumtemperatur. Danach gibt man 13.8 g (0.3 Mol) Methylhydrazin und weitere 30.3 g (0.3 Mol) Triethylamin zu, rührt eine Stunde bei 60°C, filtriert ausgefallenes Salz ab, engt das Filtrat im Vakuum ein und chromatographiert den Rückstand an Kieselgel (Laufmittel: Dichlormethan).

Man erhält 53.1 g (54% der Theorie) an N-[5-(3,4-Dichlorphenyl)-1-methylpyrazol-3-yl]-N-methylamino-essigsäuremethylester als Öl.

¹H-NMR (CDCl₃/Tetramethylsilan): δ = 2.88 (3H); 3.75 (5H); 3.79 (3H); 6.18 (1H); 7.4-7.45 (1H); 7.5-7.6 (1H); 7.84 (1H) ppm.

### Beispiel 5:

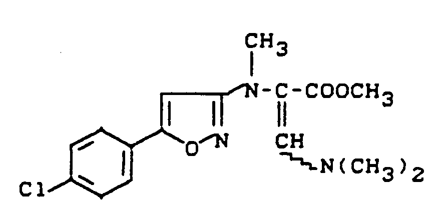

9.5 g (0.034 Mol) N-[5-(4-Chlorphenyl)-isoxazol-3-yl)-N-methylaminoessigsäuremethylester werden zusammen mit 9.1 g (0.052 Mol) t-Butoxy-bis-(dimethylamino)-methan bei einer Badtemperatur von 100°C eine Stunde lang erhitzt. Zur Aufarbeitung wird im Wasserstrahlvakuum eingeengt und der Rückstand aus Chloroform/Toluol (8:3) umkristallisiert.

Man erhält 8.5 g (75% der Theorie) an 2-{N-[5-(4-Chlorphenyl)-isoxazol-3-yl]-N-methylamino}-3-dimethylaminoacrylsäuremethylester vom Schmelzpunkt 190°C.

### Beispiel V-5:

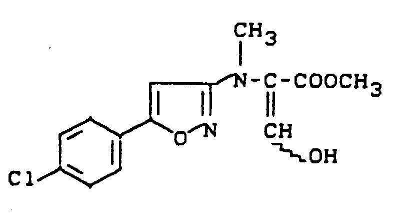

5 g (0.0149 Mol) 2-{N-[5-(4-Chlorphenyl)-isoxazol-3-yl)-N-methylamino}-3-dimethylaminoacrylsäuremethylester und 15 ml (0.015 Mol) 1 normale Salzsäure werden in 20 ml Dimethylformamid 2 Stunden bei Raumtemperatur gerührt, anschließend mit Wasser versetzt, mit Dichlormethan extrahiert, die organischen Phasen über Natriumsulfat getrocknet und im Vakuum eingeengt.

Man erhält 4.1 g (89% der Theorie) an 2-{N-[5-(4-Chlorphenyl)-isoxazol-3-yl]-N-methylamino]-3-hydroxyacrylsäuremethylester als öliges Rohprodukt, welches ohne weitere Reinigung in die nächste Stufe eingesetzt werden kann.

### Beispiel II-5:

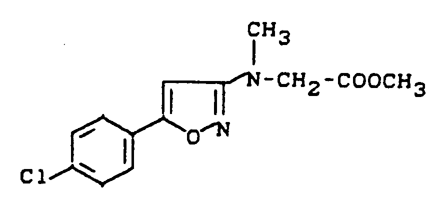

Zu 23.6 g (0.1 Mol) (4-Chlorphenyl)-(2,2-dichlorvinyl)keton (vgl. Synthesis 1982, 204) in 100 ml Tetrahydrofuran gibt man tropfenweise unter Rühren bei 20°C eine Mischung aus 10.3 g (0.1 Mol) Sarcosinmethylester und 10.1 g (0.1 Mol) Triethylamin zu, rührt eine Stunde bei Raumtemperatur, filtriert ausgefallenes Salz ab und gießt das Filtrat in eine Mischung aus 16 g (0.23 Mol) Hydroxylaminhydrochlorid und 200 ml Pyridin. Man rührt eine Stunde bei 80°C, gibt etwas Toluol zu, destilliert Toluol und Pyridin gemeinsam ab, verteilt den Rückstand zwischen Dichlormethan und Wasser, trennt die organische Phase ab, trocknet über Natriumsulfat, engt im Vakuum ein und kristallisiert den Rückstand aus 450 ml Methanol um.

Man erhält 13.9 g (50% der Theorie) an N-[5-(4-Chlorphenyl)-isoxazol-3-yl]-N-methylamino-essigsäuremethylester vom Schmalzpunkt 141°C.

### Beispiel 6:

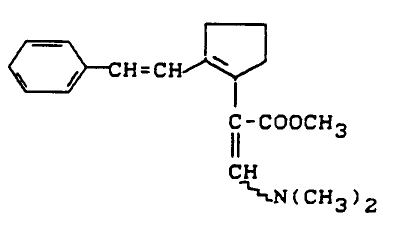

Zu 9.5 g (0.039 Mol) 2-[2-(2-Phenylethenyl)-1-cyclopentenyl)-essigsäuremethylester (vgl. DE-OS 3928999) in 40 ml niedrig siedendem Petrolether gibt man bei Raumtemperatur tropfenweise unter Rühren 9.5 g (0.054 Mol) t-Butoxy-bis-(dimethylamino)-methan und rührt anschließend weitere 12 Stunden bei Raumtemperatur. Zur Aufarbeitung wird ausgefallenes Produkt abgesaugt und getrocknet.

Man erhält 8.5 g (73% der Theorie) an 2-[2-(2-Phenylethenyl)-1-cyclopentenyl]-3-dimethylaminoacrylsäuremethylester als Öl.

¹H-NMR (CDCl₃/Tetramethylsilan):
- δ =: 7.56 (1H); 7.12-7.42 m (5H); 6.80, 6.85 d (1H); 6.38, 6.43 d (1H); 3.66 (3H); 2.83 (6H); 2.5-3.0 m (4H); 2.8-2.08 (2H) ppm.

### Beispiel V-6:

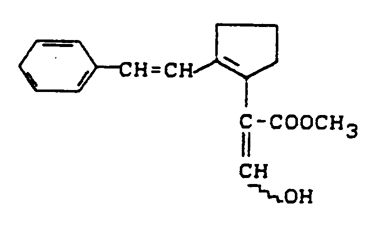

Zu 8.5 g (0.0286 Mol) 2-[2-(2-Phenylethenyl)-1-cyclopentenyl]-3-dimethylamino-acrylsäuremethylester in 38 ml Dimethylformamid gibt man tropfenweise unter Rühren 25 ml 2 normale Salzsäure, rührt anschließend 1 Stunde bei Raumtemperatur, verdünnt dann die Mischung mit Wasser und extrahiert mit Diethylether. Die vereinigten Etherphasen werden über Natriumsulfat getrocknet und im Vakuum eingeengt.

Man erhält 7.1 g (92% der Theorie) an 2-[2-(2-Phenylethenyl)-1-cyclopentenyl]-3-hydroxyacrylsäuremethylester als Öl, welches ohne weitere Reinigung in die nächste Stufe eingesetzt werden kann.

¹H-NMR (CDCl₃/Tetramethylsilan):
- δ =: 12.0 d (1H); 7.03-7.45 m (5H); 6.93, 6.99 d (1H); 6.45, 6.51 d (1H); 3.79 (3H); 2.55-2.75 m (4H); 1.85-2.05 m (2H) ppm.

### Beispiel IV-6:

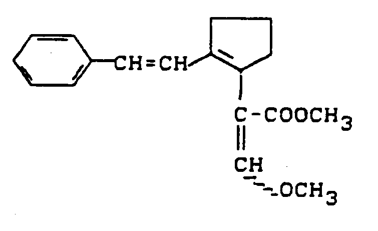

Zu 7.1 g (0.026 Mol) 2-[2-(2-Phenylethenyl)-1-cyclopentenyl]-3-hydroxyacrylsäuremethylester und 7.3 g (0.053 Mol) Kaliumcarbonat in 30 ml Dimethylformamid gibt man bei 0°C tropfenweise unter Rühren 3.3 g (0.026 Mol) Dimethylsulfat, rührt anschließend 15 Stunden bei Raumtemperatur, gießt dann die Reaktionsmischung in Wasser, extrahiert mit Diethylether, trocknet die vereinigten organischen Phasen über Natriumsulfat, engt im Vakuum ein und kristallisiert den Rückstand aus Toluol um.

Man erhält 4.6 g (62% der Theorie) an 2-[2-(2-Phenylethenyl)-1-cyclopentenyl]-3-methoxy-acrylsäuremethylester vom Schmalzpunkt 123°-125°C.

### Beispiel 7:

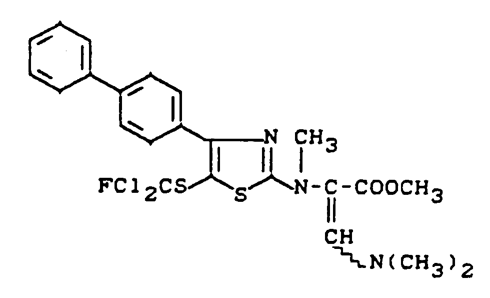

Ein Gemisch aus 6.0 g (0.013 Mol) N-[5-Dichlorfluormethylthio-4-(4-phenyl)-phenylthiazol-2-yl]-N-methylaminoessigsäuremathylester und 20 g (0.124 Mol) t-Butoxy-bis-(dimethylamino)-methan wird für 10 Stunden auf 100°C erwärmt, anschließend abgekühlt, mit 200 ml Wasser und 200 ml Ethylacetat versetzt. Die Ethylacetatphase wird abgetrennt, getrocknet und eingeengt.

Man erhält 6.2 g (91% der Theorie) an 2-{N-[5-Dichlorfluormethylthio-4-(4-phenyl)-phenylthiazol-2-yl]-N-methylamino}-3-dimethylaminoacrylsäuremethylester als Öl.

¹H-NMR (DMSO-d₆/Tetramethylsilan):
- δ =: 3.25 (3H; N-CH₃); 3.60 (3H; COOCH₃); 3.34 (6H; N(CH₃)₂) ppm.

### Beispiel IV-7:

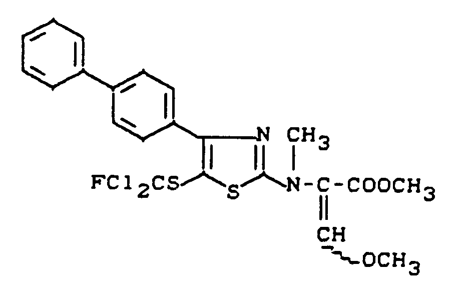

Zu 5.8 g (0.011 Mol) 2-{N-[5-Dichlorfluormethylthio-4-(4-phenyl)-phenylthiazol-2-yl)-N-methylamino}-3-dimethylaminoacrylsäuremethylester in 200 ml Dimethylformamid gibt man 2.5 ml (0.025 Mol) konzentrierte Salzsäure, erhitzt für 3 Stunden auf 60°C, kühlt dann ab, gibt 6.9 g (0.05 Mol) Kaliumcarbonat und 6.3 g (0.05 Mol) Dimethylsulfat zu, rührt 12 Stunden bei Raumtemperatur, gießt anschließend die Reaktionsmischung in Wasser, extrahiert mit Dichlormethan, trocknet die organische Phase über Natriumsulfat, engt im Vakuum ein und chromatographiert den Rückstand an Kieselgel (Laufmittel: Dichlormethan).

Man erhält 3.2 g (60% der Theorie) an 2-{N-[5-Dichlorfluormethylthio-4-(4-phenyl)-phenylthiazol-2-yl]-N-methylamino}-3-methoxyacrylsäuremethylester als Öl.

¹H-NMR (CDCl₃/Tetramethylsilan):
- δ =: 3.34 (3H; N-CH₃); 3.37 (3H; COOCH₃); 3.78 (3H; OCH₃) ppm.

### Beispiel 8:

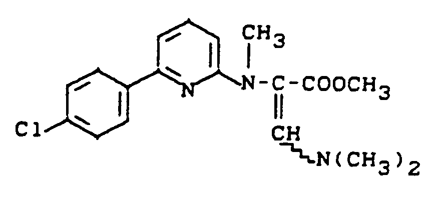

403 g (1.386 Mol) N-[6-(4-Chlorphenyl)-2-pyridyl]-N-methylaminoessigsäuremethylester und 981.4 g (5.63 Mol) t-Butoxy-bis-(dimethylamino)-methan werden 25 Stunden bei 60°C gerührt, anschließend im Vakuum eingeengt, der Rückstand mit 3 l Dichlormethan versetzt, dreimal mit jeweils 500 ml Wasser gewaschen, über Magnesiumsulfat getrocknet, im Vakuum eingeengt, der Rückstand mit 2 l Petrolether verrührt, abgesaugt, mit 1 l Petrolether nachgewaschen und an der Luft getrocknet.

Man erhält 452 g (89% der Theorie) an 2-{N-[6-(4-Chlorphenyl)-2-pyridyl]-N-methylamino}-3-dimethylaminoacrylsäuremethylester vom Schmelzpunkt 101°C.

### Beispiel IV-8:

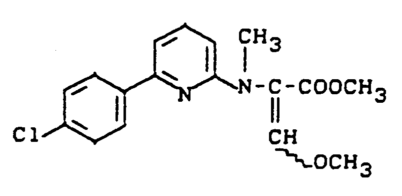

400 g (1.16 Mol) 2-{N-[6-(4-Chlorphenyl)-2-pyridyl]-N-methylamino}-3-dimethylaminoacrylsäuremethylester, 1158 ml 2 normale Salzsäure und 1158 ml Wasser in 4 l Dimethylformamid werden 2 Stunden bei 60°C gerührt, nach Abkühlen auf 20°C in 15 l Wasser und 250 g Natriumhydrogencarbonat eingerührt, ausgefallenes Produkt abgesaugt, in 2 l Dichlormethan gelöst, dreimal mit jeweils 1 l Wasser gewaschen, über Magnesiumsulfat getrocknet und im Vakuum eingeengt. Der Rückstand wird in 4 l Dimethylformamid gelöst, mit 320 g (2.3 Mol) Kaliumcarbonat versetzt, 30 Minuten bei Raumtemperatur gerührt, tropfenweise unter Kühlung mit 178 g (1.4 Mol) Dimethylsulfat versetzt, in 15 l Wasser eingerührt, zweimal mit jeweils 1 l Dichlormethan extrahiert, die Dichlormethanphasen zweimal mit jeweils 500 ml Wasser gewaschen, über Magnesiumsulfat getrocknet und im Vakuum eingeengt. Der Rückstand wird aus 600 ml t-Amyl-methyl-ether umkristallisiert und im Vakuum bei 40°C getrocknet.

Man erhält 270 g (70% der Theorie) an 2-{N-[6-(4-Chlorphenyl)-2-pyridyl]-N-methylamino}-3-methoxyacrylsäuremethylester vom Schmelzpunkt 135°C.

### Beispiel II-8:

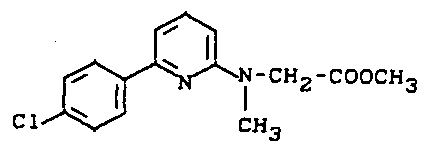

123 g (0.444 Mol) N-[6-(4-Chlorphenyl)-2-pyridyl]-N-methylaminoessigsäure und 1 ml konzentrierte Schwefelsäure werden in 750 ml Methanol 16 Stunden auf Rückflußtemperatur erhitzt, anschließend im Vakuum eingeengt, der Rückstand in 500 ml Dichlormethan gelöst, einmal mit 250 ml 3%iger wäßriger Natriumhydroxidlösung und dreimal mit jeweils 100 ml Wasser gewaschen, über Magnesiumsulfat getrocknet, im Vakuum eingeengt und der Rückstand mit 500 ml Petrolether verrührt, abgesaugt und getrocknet.

Man erhält 92 g (71% der Theorie) an N-[6-(4-Chlorphenyl)-2-pyridyl]-N-methylaminoessigsäuremethylester vom Schmelzpunkt 54°C.

### Beispiel 9:

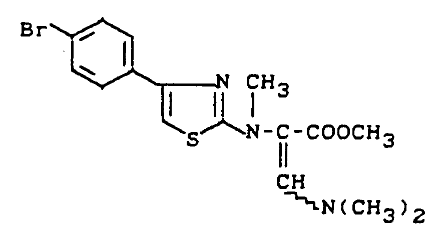

17 g (0.04982 Mol) N-[4-(4-Bromphenyl)-2-thiazolyl]-N-methylamino-essigsäuremethylester und 17.4 g (0.1078 Mol) t-Butoxy-bis-(dimethylamino)-methan werden 6 Stunden bei 100°C gerührt, abgekühlt, mit Eiswasser versetzt, einige Stunden bei Raumtemperatur gerührt, ausgefallene Kristalle abgesaugt und getrocknet.

Man erhält 17.5 g (89% der Theorie) an 2-{N-[4-(4-Bromphenyl)-2-thiazolyl]-N-methylamino}-3-dimethylaminoacrylsäuremethylester vom Schmelzpunkt 184-185°C.

### Beispiel 10:

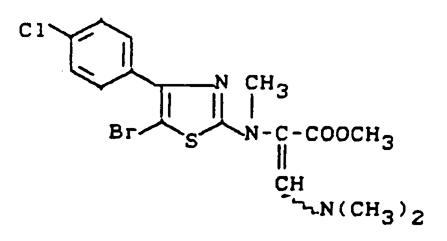

3.75 g (0.00998 Mol) N-[5-Brom-4-(4-chlorphenyl)-2-thiazolyl]-N-methylamino-essigsäuremethylester und 3.5 g (0.02168 Mol) t-Butoxy-bis-(dimethylamino)-methan in 10 ml Dioxan werden 15 Stunden bei Rückflußtemperatur gerührt, abgekühlt, mit Wasser und Dichlormethan versetzt, 2 Stunden bei Raumtemperatur gerührt, die organische Phase abgetrennt, über Magnesiumsulfat getrocknet und im Vakuum eingeengt.

Man erhält 4.1 g (95% der Theorie) an 2-{N-[5-Brom-4-(4-chlorphenyl)-2-thiazolyl]-N-methylamino}-3-dimethylaminoacrylsäuremethylester als Öl.

¹H-NMR (CDCl₃/Tetramethylsilan):
- δ =: 3.24 (3H; N-CH₃); 3.69 (3H; COOCH₃); 3.70 (6H; N(CH₃)₂) ppm.

### Vergleichsversuch A: (vgl. Herstellungsbeispiel 2)

Zu einem Gemisch aus 1.2 g (0.05 Mol) Natriumhydrid in 30 ml Dimethylformamid gibt man eine Lösung von 5.2 g (0.021 Mol) N-(6-Brom-2-pyridyl)-N-methylaminoessigsäuremethylester und 30 g (0.72 Mol) Ameisensäuremethylester in 30 ml Dimethylformamid, rührt anschließend 15 Stunden bei Raumtemperatur, gibt dann 12.5 g (0.1 Mol) Dimethylsulfat zu, rührt weitere 15 Stunden bei Raumtemperatur, gießt dann die Mischung in Wasser, extrahiert mit Ethylacetat, trocknet über Magnesiumsulfat, engt im Vakuum ein und chromatographiert den Rückstand an Kieselgel (Laufmittel: Dichlormethan/n-Hexan 5:1).

Man erhält als 1. Fraktion:
1.5 g (29% der Theorie) an Ausgangsprodukt N-(6-Brom-2-pyridyl)-N-methylaminoessigsäuremethylester
und als 2. Fraktion:
1.8 g (30% der Theorie) an 2-[N-(6-Brom-2-pyridyl)-N-methylamino]-3-methoxyacrylsäuremethylester vom Schmelzpunkt 78-79°C.

### Vergleichsversuch B: (vgl. Herstellungsbeispiel 2)

2.6 g (0.01 Mol) N-(6-Brom-2-pyridyl)-N-methylaminoessigsäuremethylester und 2.4 g (0.02 Mol) Dimethylformamiddimethylacetal entweder gelöst in 5 ml Toluol oder ohne Zusatz eines Verdünnungsmittels wurden 15 Stunden bei Rückflußtemperatur gerührt. Eine dünnschichtchromatographische Untersuchung dar Reaktionsgemische ergab in beiden Fällen nur Spuren des gewünschten Reaktionsproduktes 2-[N-(6-Brom-2-pyridyl)-N-methylamino]-3-methoxyacrylsäuremethylester.

### Vergleichsversuch C: (vgl. Herstellungsbeispiel 7)

6.45 g (0.015 Mol) N-[5-Dichlorfluormethylthio-4-(4-phenyl)-phenyl-2-thiazolyl]-N-methylaminoessigsäuremethylester und 20 g (0.48 Mol) Ameisensäuremethylester in 10 ml Dimethylformamid werden bei 0°C tropfenweise unter Rühren zu 1 g (0.033 Mol) Natriumhydrid in 10 ml Dimethylformamid gegeben. Man rührt anschließend 15 Stunden bei Raumtemperatur, gibt dann 4.0 g (0.032 Mol) Dimethylsulfat zu, rührt weitere 3 Stunden bei Raumtemperatur, gießt dann das Reaktionsgemisch in Wasser, extrahiert mit Ethylacetat, trocknet über Natriumsulfat und engt im Vakuum ein.

Der Rückstand enthält nur Spuren der gewünschten Verbindung 2-{N-[5-Dichlorfluormethylthio-4-(4-phenyl)-phenyl-2-thiazolyl]-N-methylamino}-3-methoxyacrylsäuremethylester.

### Vergleichsversuch D: (vgl. Herstellungsbeispiel 8)

Zu 2.4 g (0.1 Mol) Natriumhydrid in 60 ml Dimethylformamid gibt man tropfenweise unter Rühren bei Raumtemperatur 10.3 g (0.035 Mol) N-[6-(4-Chlorphenyl)-2-pyridyl]-N-methylaminoessigsäuremethylester und 50 g (1.2 Mol) Ameisensäuremethylester in 50 ml Dimethylformamid, rührt anschließend 3 Tage bei Raumtemperatur, gibt weitere 0.5 g (0.021 Mol) Natriumhydrid und 10 g (0.24 Mol) Ameisensäuremethylester zu, rührt weitere 5 Stunden bei Raumtemperatur, gibt danach abermals 0.5 g (0.021 Mol Natriumhydrid und 10 g (0.24 Mol) Ameisensäuremethylester zu und rührt weitere 24 Stunden bei Raumtemperatur.

Danach gibt man 14.0 g (0.11 Mol) Dimethylsulfat zu und rührt weitere 48 Stunden bei Raumtemperatur. Zur Aufarbeitung gießt man die Reaktionsmischung in Wasser, extrahiert mit Ethylacetat, trocknet über Magnesiumsulfat, engt im Vakuum ein und chromatographiert den Rückstand an Kieselgel (Laufmittel: Dichlormethan/n-Hexan 5:1).

Man erhält als 1. Fraktion
7.1 g (69% der Theorie) an Ausgangsverbindung N-[6-(4-Chlorphenyl)-2-pyridyl]-N-methylaminoessigsäuremethylester
und als 2. Fraktion
0.5 g (4.2% der Theorie) an gewünschtem Produkt 2-{N-[6-(4-Chlorphenyl)-2-pyridyl]-N-methylamino}-3-methoxyacrylsäuramethylester vom Schmelzpunkt 120°C.

## Patentansprüche

1. Verfahren zur Herstellung von 3-Aminoacrylestern der allgemeinen Formel (I), 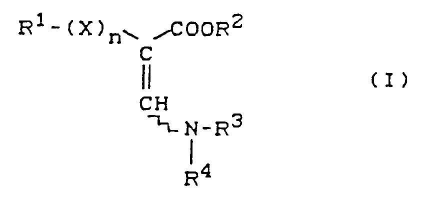 in welcher
R¹ für einen gegebenenfalls einfach oder mehrfach, gleich oder verschieden substituierten, gesättigten, einfach oder mehrfach ungesättigten oder aromatischen Carbocyclus mit 5 bis 10 Kohlenstoffatomen oder für einen gegebenenfalls einfach oder mehrfach, gleich oder verschieden substituierten, gesättigten, einfach oder mehrfach ungesättigten oder aromatischen Heterocyclus mit 2 bis 9 Kohlenstoffatomen und 1 bis 5 gleichen oder verschiedenen Heteroatomen - insbesondere Stickstoff, Sauerstoff oder Schwefel - steht, wobei als Substituenten jeweils infrage kommen:
Halogen, Cyano, Nitro, jeweils geradkettiges oder verzweigtes Alkyl, Alkoxy oder Alkylthio mit jeweils 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl, Alkoximinoalkyl, Dialkylamino oder Dialkylaminocarbonyl mit jeweils 1 bis 4 Kohlenstoffatomen in den einzelnen Alkylteilen, jeweils geradkettiges oder verzweigtes Alkenyl oder Alkinyl mit jeweils 2 bis 8 Kohlenstoffatomen, Cycloalkyl mit 3 bis 7 Kohlenstoffatomen, zweifach verknüpftes Alkandiyl mit 3 bis 5 Kohlenstoffatomen, oder jeweils im Arylteil oder im Heteroarylteil gegebenenfalls einfach oder mehrfach, gleich oder verschieden
- durch Halogen, Alkyl, Alkoxy, Alkylthio, Halogenalkyl, Halogenalkoxy, Halogenalkylthio, Dioxyalkylen, Halogen-substituiertes Dioxyalkylen oder gegebenenfalls substituiertes Phenyl
substituiertes Aryl, Aryloxy, Arylthio, Arylcarbonyl, Aralkyl, Aralkenyl, Aralkinyl, Aralkyloxy, Aralkylthio, Heteroarylalkyl, Heteroarylalkenyl, Heteroaryloxy, Heteroarylthio, Heteroarylcarbonyl, Heteroarylalkyloxy, Heteroarylalkylthio oder Heteroaryl mit jeweils 6 bis 10 Kohlenstoffatomen im Arylteil oder mit 2 bis 9 Kohlenstoffatomen und 1 bis 4 gleichen oder verschiedenen Heteroatomen - insbesondere Stickstoff, Sauerstoff und/oder Schwefel - im Heteroarylteil und gegebenenfalls 1 bis 6 Kohlenstoffatomen im geradkettigen oder verzweigten Alkylteil oder gegebenenfalls 2 bis 6 Kohlenstoffatomen im geradkettigen oder verzweigten Alkenylteil bzw. Alkinylteil;
R² für geradkettiges oder verzweigtes Alkyl mit 1 bis 6 Kohlenstoffatomen oder für gegebenenfalls im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 9 Kohlenstoffatomen und 1 bis 4 gleichen oder verschiedenen Heteroatomen - insbesondere Stickstoff, Sauerstoff und/oder Schwefel - im Heteroarylteil und gegebenenfalls 1 bis 4 Kohlenstoffatomen im geradkettigen oder verzweigten Alkylteil;
R³ und R⁴ entweder unabhängig voneinander jeweils für geradkettiges oder verzweigtes Alkyl mit 1 bis 6 Kohlenstoffatomen stehen oder gemeinsam mit dem Stickstoffatom, an welches sie gebunden sind, für einen gegebenenfalls einfach oder mehrfach, gleich oder verschieden substituierten, gesättigten 5- bis 7-gliedrigen Heterocyclus stehen, der gegebenenfalls ein weiteres Heteroatom - insbesondere Stickstoff, Sauerstoff oder Schwefel - enthalten kann, wobei als Substituenten geradkettige oder verzweigte Alkylreste mit 1 bis 4 Kohlenstoffatomen infrage kommen,
X für Sauerstoff, Schwefel oder für einen der Reste 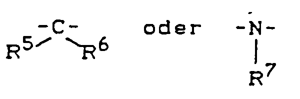 steht und
n für eine Zahl 0 oder 1 steht, wobei
R⁵, R⁶ und R⁷ unabhängig voneinander jeweils für Wasserstoff, für jeweils geradkettiges oder verzweigtes Alkyl mit 1 bis 6 Kohlenstoffatomen, Alkenyl mit 2 bis 6 Kohlenstoffatomen oder für jeweils gegebenenfalls im Arylteil einfach oder mehrfach, gleich oder verschieden substituiertes Aralkyl mit 1 bis 6 Kohlenstoffatomen im geradkettigen oder verzweigten Alkylteil und 6 bis 10 Kohlenstoffatomen im Arylteil oder Aralkenyl mit 2 bis 6 Kohlenstoffatomen im geradkettigen oder verzweigten Alkenylteil und 6 bis 10 Kohlenstoffatomen im Arylteil stehen, wobei als Arylsubstituenten jeweils die bei R² genannten infrage kommen,
dadurch gekennzeichnet, daß man substituierte Essigsäureester der Formel (II),
R¹-(X)ₙ-CH₂-COOR² (II)
in welcher
R¹, R², X und n die oben angegebene Bedeutung haben,
mit Orthoameisensäurediamidestern der Formel (III), 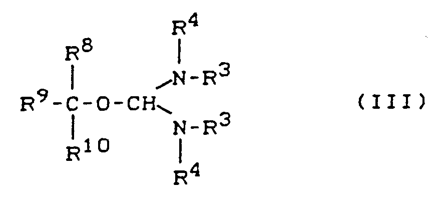 in welcher
R⁸ für Wasserstoff oder Alkyl steht,
R⁹ und R¹⁰ entweder unabhängig voneinander jeweils für Alkyl stehen oder gemeinsam mit dem Kohlenstoffatom, an welches sie gebunden sind, für einen Cycloalkylrest stehen und
R³ und R⁴ die oben angegebene Bedeutung haben,
gegebenenfalls in Gegenwart eines Verdünnungsmittels umsetzt.

2. Verfahren gemäß Anspruch 1, dadurch gekennzeichnet, daß man bei Temperaturen von -35°C bis +150°C arbeitet.

3. Verfahren gemäß Anspruch 1, dadurch gekennzeichnet, daß man pro Mol an substituiertem Essigsäureester der Formel (II) 1,0 bis 15,0 Mol an Orthoameisensäurediamidester der Formel (III) einsetzt.

4. Verfahren gemäß Anspruch 1, dadurch gekennzeichnet, daß man Orthoameisensäurediamidester der Formel (III) einsetzt, in denen
R³ und R⁴ die in Anspruch 2 angegebene Bedeutung haben und
R⁸ für Wasserstoff oder für geradkettiges oder verzweigtes Alkyl mit 1 bis 4 Kohlenstoffatomen steht, und
R⁹ und R¹⁰ unabhängig voneinander jeweils für geradkettiges oder verzweigtes Alkyl mit 1 bis 4 Kohlenstoffatomen oder gemeinsam mit dem Kohlenstoffatom, an welches sie gebunden sind, für Cycloalkyl mit 5 bis 7 Kohlenstoffatomen stehen.

## Claims

1. Process for the preparation of 3-aminoacrylic esters of the general formula (I) 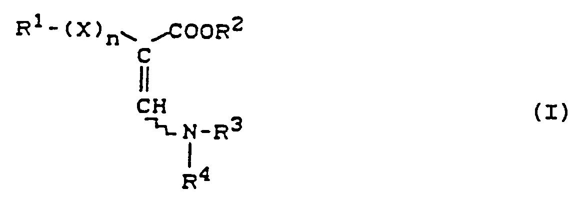 in which
R¹ represents a carbocycle which has 5 to 10 carbon atoms and which is optionally monosubstituted or polysubstituted by identical or different substituents, saturated, monounsaturated or polyunsaturated or aromatic, or represents a heterocycle which has 2 to 9 carbon atoms and 1 to 5 identical or different hetero atoms - in particular nitrogen, oxygen or sulphur - and which is optionally monosubstituted or polysubstituted by identical or different substituents, saturated, monounsaturated or polyunsaturated or aromatic, suitable substituents in each case being:
halogen, cyano, nitro, in each case straight-chain or branched alkyl, alkoxy or alkylthio, each of which has 1 to 6 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl, alkoximinoalkyl, dialkylamino or dialkylaminocarbonyl, each of which has 1 to 4 carbon atoms in the individual alkyl moieties, in each case straight-chain or branched alkenyl or alkinyl, each of which has 2 to 8 carbon atoms, cycloalkyl having 3 to 7 carbon atoms, divalent alkanediyl having 3 to 5 carbon atoms, or aryl, aryloxy, arylthio, arylcarbonyl, aralkyl, aralkenyl, aralkinyl, aralkyloxy, aralkylthio, heteroarylalkyl, heteroarylalkenyl, heteroaryloxy, heteroarylthio, heteroarylcarbonyl, heteroarylalkyloxy, heteroarylalkylthio or heteroaryl, each of which has 6 to 10 carbon atoms in the aryl moiety or 2 to 9 carbon atoms and 1 to 4 identical or different hetero atoms - in particular nitrogen, oxygen and/or sulphur - in the heteroaryl moiety and if appropriate 1 to 6 carbon atoms in the straight-chain or branched alkyl moiety or if appropriate 2 to 6 carbon atoms in the straight-chain or branched alkenyl moiety or alkinyl moiety, each of which is optionally monosubstituted or polysubstituted in the aryl moiety or in the heteroaryl moiety by identical or different substituents from the series comprising
- halogen, alkyl, alkoxy, alkylthio, halogenoalkyl, halogenoalkoxy, halogenoalkylthio, dioxyalkylene, halogensubstituted dioxyalkylene or optionally substituted phenyl;
R² represents straight-chain or branched alkyl having 1 to 6 carbon atoms, or represents aralkyl which has 1 to 4 carbon atoms in the straight-chain or branched alkyl moiety and 6 to 10 carbon atoms in the aryl moiety and which is optionally monosubstituted or polysubstituted in the aryl moiety by identical or different substituents, suitable aryl substituents being: halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 8 carbon atoms in the individual alkyl moieties, cycloalkyl having 3 to 7 carbon atoms, divalent alkanediyl having 3 to 5 carbon atoms, or aryl, aralkyl, aryloxy or aralkyloxy, each of which has 6 to 10 carbon atoms in the aryl moiety and if appropriate 1 to 4 carbon atoms in the straight-chain or branched alkyl moiety and each of which is optionally monosubstituted or polysubstituted in the aryl moiety by identical or different substituents from the series comprising halogen, alkyl, alkoxy, alkylthio, halogenoalkyl, halogenoalkoxy or halogenoalkylthio, each of which has 1 to 4 carbon atoms and if appropriate 1 to 9 identical or different halogen atoms, or heteroarylalkyl or heteroaryl, each of which has 2 to 9 carbon atoms and 1 to 4 identical or different hetero atoms, - in particular nitrogen, oxygen and/or sulphur - in the heteroaryl moiety and if appropriate 1 to 4 carbon atoms in the straight-chain or branched alkyl moiety and each of which is optionally monosubstituted or polysubstituted in the heteroaryl moiety by identical or different substituents from the series comprising halogen, alkyl, alkoxy, alkylthio, halogenoalkyl, halogenoalkoxy or halogenoalkylthio, each of which has 1 to 4 carbon atoms and if appropriate 1 to 9 identical or different halogen atoms;
R³ and R⁴ either independently of one another in each case represent straight-chain or branched alkyl having 1 to 6 carbon atoms, or together with the nitrogen atom to which they are bonded represent a saturated 5- to 7-membered heterocycle which can optionally contain a further hetero atom - in particular nitrogen, oxygen or sulphur - and which is optionally monosubstituted or polysubstituted by identical or different substituents, suitable substituents being straight-chain or branched alkyl radicals having 1 to 4 carbon atoms,
X represents oxygen, sulphur or one of the radicals 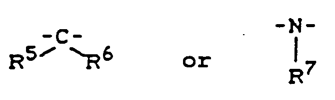 and
n represents a number 0 or 1 where
R⁵, R⁶ and R⁷ independently of one another in each case represent hydrogen, in each case straight-chain or branched alkyl having 1 to 6 carbon atoms or alkenyl having 2 to 6 carbon atoms, or represent aralkyl which has 1 to 6 carbon atoms in the straight-chain or branched alkyl moiety and 6 to 10 carbon atoms in the aryl moiety or aralkenyl which has 2 to 6 carbon atoms in the straight-chain or branched alkenyl moiety and 6 to 10 carbon atoms in the aryl moiety, each of these aralkyl or aralkenyl radicals optionally being monosubstituted or polysubstituted in the aryl moiety by identical or different substituents, suitable aryl substituents in each case being those mentioned in the case of R²,
characterised in that substituted acetic esters of the formula (II)
R¹-(X)ₙ-CH₂-COOR² (II)
in which
R¹, R², X and n have the abovementioned meaning, are reacted with orthoformic acid diamide esters of the formula (III) 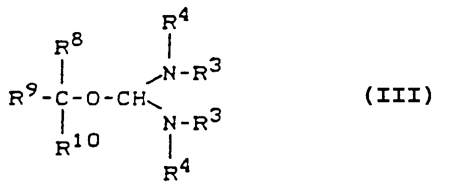 in which
R⁸ represents hydrogen or alkyl,
R⁹ and R¹⁰ either independently of one another in each case represent alkyl, or together with the carbon atom to which they are bonded represent a cycloalkyl radical, and
R³ and R⁴ have the abovementioned meaning,
if appropriate in the presence of a diluent.

2. Process according to Claim 1, characterised in that it is carried out at temperatures from -35°C to +150°C.

3. Process according to Claim 1, characterised in that 1.0 to 15.0 mol of orthoformic acid diamide ester of the formula (III) are employed per mole of substituted acetic ester of the formula (II).

4. Process according to Claim 1, characterised in that orthoformic acid diamide esters of the formula (III) are employed in which
R³ and R⁴ have the meaning given in Claim 2 and
R⁸ represents hydrogen or straight-chain or branched alkyl having 1 to 4 carbon atoms, and
R⁹ and R¹⁰ independently of one another in each case represent straight-chain or branched alkyl having 1 to 4 carbon atoms, or together with the carbon atom to which they are bonded represent cycloalkyl having 5 to 7 carbon atoms.

## Revendications

1. Procédé pour la préparation d'esters 3-aminoacryliques répondant à la formule générale (I) 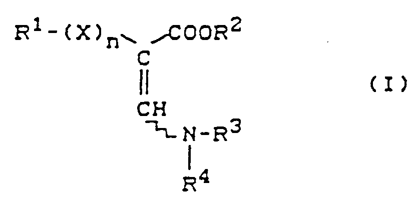 dans laquelle
R¹ représente un radical carbocyclique contenant de 5 à 10 atomes de carbone, saturé, une fois ou plusieurs fois insaturé ou encore aromatique, éventuellement substitué une ou plusieurs fois de manière identique ou différente, ou encore un radical hétérocyclique saturé, une ou plusieurs fois insaturé ou encore aromatique, contenant de 2 à 9 atomes de carbone et de 1 à 5 hétéroatomes identiques ou différents - en particulier un atome d'azote, un atome d'oxygène ou un atome de soufre - éventuellement substitué une ou plusieurs fois de manière identique ou différente, dans lesquels, comme substituants, on envisage respectivement :
un atome d'halogène, un groupe cyano, un groupe nitro; un groupe alkyle, un groupe alcoxy ou un groupe alkylthio contenant respectivement de 1 à 6 atomes de carbone, chacun de ces groupes étant à chaîne droite ou ramifiée; un groupe halogénalkyle, un groupe halogénalcoxy ou un groupe halogénalkylthio contenant respectivement de 1 à 4 atomes de carbone et de 1 à 9 atomes d'halogène identiques ou différents, chacun de ces groupes étant à chaîne droite ou ramifiée; un groupe alcoxycarbonyle, un groupe alcoximinoalkyle, un groupe dialkylamino ou dialkylaminocarbonyle contenant respectivement de 1 à 4 atomes de carbone dans les fractions alkyle individuelles, chacun de ces groupes étant à chaîne droite ou ramifiée; un groupe alcényle ou un groupe alcynyle contenant respectivement de 2 à 8 atomes de carbone, chacun de ces groupes étant à chaîne droite ou ramifiée; un groupe cycloalkyle contenant de 3 à 7 atomes de carbone; un groupe alcanediyle à double liaison contenant de 3 à 5 atomes de carbone; ou représente un groupe aryle, un groupe aryloxy, un groupe arylthio, un groupe arylcarbonyle, un groupe aralkyle, un groupe aralcényle, un groupe aralcynyle, un groupe aralcoxy, un groupe aralkylthio, un groupe hétéroarylalkyle, un groupe hétéroarylalcényle, un groupe hétéroaryloxy, un groupe hétéroarylthio, un groupe hétéroarylcarbonyle, un groupe hétéroarylalcoxy, un groupe hétéroarylalkylthio ou un groupe hétéroaryle, contenant respectivement de 6 à 10 atomes de carbone dans la fraction aryle ou encore contenant de 2 à 9 atomes de carbone et de 1 à 4 hétéroatomes identiques ou différents - en particulier, un atome d'azote, un atome d'oxygène et/ou un atome de soufre - dans la fraction hétéroaryle et éventuellement de 1 à 6 atomes de carbone dans la fraction alkyle à chaîne droite ou ramifiée ou éventuellement de 2 à 6 atomes de carbone dans la fraction alcényle, respectivement dans la fraction alcynyle à chaîne droite ou ramifiée, chacun de ces groupes portant éventuellement, dans la fraction aryle ou dans la fraction hétéroaryle, un ou plusieurs substituants identiques ou différents :
halogéno, alkyle, alcoxy, alkylthio, halogénalkyle, halogénalcoxy, halogénalkylthio, dioxyalkylène, dioxyalkylène portant un ou plusieurs substituants halogéno identiques ou différents, ou encore phényle éventuellement substitué une ou plusieurs fois de manière identique ou différente;
R² représente un groupe alkyle à chaîne droite ou ramifiée contenant de 1 à 6 atomes de carbone; ou encore un groupe aralkyle contenant de 1 à 4 atomes de carbone dans la fraction alkyle à chaîne droite ou ramifiée et de 6 à 10 atomes de carbone dans la fraction aryle, éventuellement substitué une ou plusieurs fois de manière identique ou différente dans la fraction aryle, dans lequel, comme substituants du groupe aryle, on envisage : un atome d'halogène, un groupe cyano, un groupe nitro; un groupe alkyle, un groupe alcoxy ou un groupe alkylthio contenant respectivement de 1 à 4 atomes de carbone, chacun de ces groupes étant à chaîne droite ou ramifiée; un groupe halogénalkyle, un groupe halogénalcoxy ou un groupe halogénalkylthio contenant respectivement de 1 à 4 atomes de carbone et de 1 à 9 atomes d'halogène identiques ou différents, chacun de ces groupes étant à chaîne droite ou ramifiée; un groupe alcoxycarbonyle ou alcoximinoalkyle contenant respectivement de 1 à 8 atomes de carbone dans les fractions alkyle individuelles, chacun de ces groupes étant à chaîne droite ou ramifiée; un groupe cycloalkyle contenant de 3 à 7 atomes de carbone; un groupe alcanediyle à double liaison contenant de 3 à 5 atomes de carbone; un groupe aryle, un groupe aralkyle, un groupe aryloxy ou un groupe aralcoxy contenant respectivement de 6 à 10 atomes de carbone dans la fraction aryle et éventuellement de 1 à 4 atomes de carbone dans la fraction alkyle à chaîne droite ou ramifiée, chacun de ces groupes portant éventuellement, dans la fraction aryle, un ou plusieurs substituants identiques ou différents halogéno, alkyle, alcoxy, alkylthio, halogénalkyle, halogénalcoxy ou halogénalkylthio contenant respectivement de 1 à 4 atomes de carbone et éventuellement de 1 à 9 atomes d'halogène identiques ou différents; ou encore un groupe hétéroarylalkyle ou un groupe hétéroaryle contenant respectivement de 2 à 9 atomes de carbone et de 1 à 4 hétéroatomes identiques ou différents - en particulier un atome d'azote, un atome d'oxygène et/ou un atome de soufre - dans la fraction hétéroaryle et éventuellement de 1 à 4 atomes de carbone dans la fraction alkyle à chaîne droite ou ramifiée, chacun de ces groupes portant éventuellement, dans la fraction hétéroaryle, un ou plusieurs substituants identiques ou différents halogéno, alkyle, alcoxy, alkylthio, halogénalkyle, halogénalcoxy ou halogénalkylthio contenant respectivement de 1 à 4 atomes de carbone et éventuellement de 1 à 9 atomes d'halogène identiques ou différents;
R³ et R⁴ représentent, soit indépendamment l'un de l'autre, respectivement un groupe alkyle à chaîne droite ou ramifiée contenant de 1 à 6 atomes de carbone, soit ensemble avec l'atome d'azote auquel ils sont liés, un radical hétérocyclique penta- à heptagonal saturé, éventuellement substitué une ou plusieurs fois de manière identique ou différente, qui peut éventuellement contenir un autre hétéroatome - en particulier un atome d'azote, un atome d'oxygène ou un atome de soufre, dans lequel, comme substituants, on envisage des radicaux alkyle à chaîne droite ou ramifiée contenant de 1 à 4 atomes de carbone,
X représente un atome d'oxygène, un atome de soufre ou encore un des radicaux 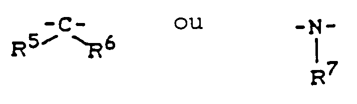
n représente le nombre 0 ou 1, dans lesquels
R⁵, R⁶ et R⁷ représentent, indépendamment l'un de l'autre, respectivement un atome d'hydrogène; un groupe alkyle contenant de 1 à 6 atomes de carbone, un groupe alcényle contenant de 2 à 6 atomes de carbone, chacun de ces groupes étant à chaîne droite ou ramifiée; ou encore un groupe aralkyle contenant de 1 à 6 atomes de carbone dans la fraction alkyle à chaîne droite ou ramifiée et de 6 à 10 atomes de carbone dans la fraction aryle, ou encore un groupe aralcényle contenant de 2 à 6 atomes de carbone dans la fraction alcényle à chaîne droite ou ramifiée et de 6 à 10 atomes de carbone dans la fraction aryle, chacun de ces groupes étant éventuellement substitué dans la fraction aryle une ou plusieurs fois de manière identique ou différente, dans lesquels, comme substituants du groupe aryle, on envisage respectivement ceux mentionnés dans R²,
caractérisé en ce qu'on fait réagir des esters acétiques substitués de formule (II)
R¹-(X)ₙ-CH₂-COOR² (II)
dans laquelle
R¹, R², X et n ont la signification indiquée ci-dessus,
avec des esters de diamides d'acide orthoformique de formule (III) 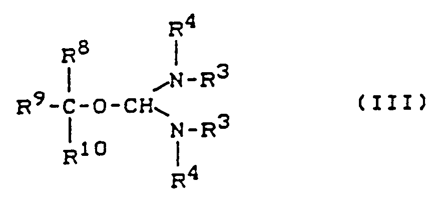 dans laquelle
R⁸ représente un atome d'hydrogène ou un groupe alkyle,
R⁹ et R¹⁰ représentent, soit indépendamment l'un de l'autre, respectivement un groupe alkyle, soit ensemble avec l'atome de carbone auquel ils sont liés, un radical cycloalkyle, et
R³ et R⁴ ont la signification indiquée ci-dessus,
éventuellement en présence d'un diluant.

2. Procédé selon la revendication 1, caractérisé en ce qu'on travaille à des températures de -35°C à +150°C.

3. Procédé selon la revendication 1, caractérisé en ce que, par mole d'ester acétique substitué de formule (II), on met en oeuvre de 1,0 à 15,0 moles d'ester de diamide d'acide orthoformique de formule (III).

4. Procédé selon la revendication 1, caractérisé en ce qu'on met en oeuvre des esters de diamides d'acide orthoformique de formule (III), dans lesquels
R³ et R⁴ ont la signification indiquée à la revendication 2, et
R⁸ représente un atome d'hydrogène ou encore un groupe alkyle à chaîne droite ou ramifiée contenant de 1 à 4 atomes de carbone, et
R⁹ et R¹⁰ représentent, indépendamment l'un de l'autre, respectivement un groupe alkyle à chaîne droite ou ramifiée contenant de 1 à 4 atomes de carbone ou, ensemble avec l'atome de carbone auquel ils sont liés, un groupe cycloalkyle contenant de 5 à 7 atomes de carbone.
